# EUROPEAN PATENT APPLICATION

(11) **EP 4 755 334 A1**
(43) Date of publication of application: **10.06.2026**
(21) Application number: 24307024.0
(22) Date of filing: 04.12.2024
(51) Int. Cl.: A61B 34/00, A61B 34/30

(54) **DEVICE FOR ORIENTING A SURGICAL TOOL**

(71) Applicant: Zentact Robotics, 38400 Saint Martin d'Hères (FR)
(72) Inventor: ARBARET, Valentin, 38400 Saint Martin d'Hères (FR); PHILBERT-ROUTIER, Damien, 38400 Saint Martin d'Hères (FR)
(74) Representative: Regimbeau

(57) **Abstract**

The present disclosure relates to a device for orienting a surgical tool (500), comprising:
- a shaft (10) extending around a shaft axis (S), the shaft (10) comprising a first winding surface (11);
- a drive system (20) configured to drive the shaft (10) in rotation around the shaft axis (S);
- a surgical tool (500) comprising a second winding surface (52);
- a rope (40) looped around the first winding surface (11) and the second winding surface (52); and
- a tensioning device tensioning the rope (40), so that a rotation of the shaft (10) rotates the rope (40) and a rotation of the rope (40) rotates the surgical tool (500) due to a friction between the rope (40) and the second winding surface (52).

## Description

### TECHNICAL FIELD

The present disclosure relates to the field of devices for orienting surgical tools, especially for otorhinolaryngology surgery.

### TECHNICAL BACKGROUND

In the current practice, otorhinolaryngology surgeons often need to manually position an endoscope to visualize the area of intervention while simultaneously operating surgical instruments with both hands. This manual approach poses significant ergonomic challenges, as the surgeon must continuously adjust the endoscope's position to maintain optimal visibility while performing precise movements with other surgical instruments. This increases complexity and workload, limiting the smoothness of movements and overall efficiency of the procedure.

An automated endoscope support system, such as described in document CN116421317A, is known. This automated endoscope support system uses integrated motors to move the endoscope, allowing the surgeon to adjust the viewing angle without direct manual manipulation and constant manual repositioning, thus with reduced workload. However, such an automated endoscope support system is complex in design, is costly and the addition of motorized mechanisms can add weight and bulk to the equipment, potentially compromising ergonomics in the operating room.

### SUMMARY

An aim of the present disclosure is to propose a device for orienting a surgical tool which is simple, easy to use and cheap.

Another aim of the present disclosure is to propose a device for orienting a surgical tool which can be used in various kinds of surgical procedures.

According to a first aspect, the present disclosure relates to a device for orienting a surgical tool, comprising:
- a shaft extending around a shaft axis, the shaft comprising a first winding surface;
- a drive system configured to drive the shaft in rotation around the shaft axis;
- a surgical tool rotationally coupled to a second winding surface;
- a rope looped around the first winding surface and the second winding surface; and
- a tensioning device tensioning the loop made by the rope around the first winding surface and the second winding surface, so that a rotation of the shaft results in a rotation of the rope and a rotation of the rope results in a rotation of the second winding surface, thus of the surgical tool, due to a friction between the rope and the second winding surface.

The device may further comprise a centering system aligning the surgical tool with a centering axis parallel to the shaft axis, wherein the surgical tool is held between the centering system and the rope due to a tension applied by the rope on the surgical tool.

The centering system may comprise a support structure extending along the centering axis, the support structure having two inclined faces located on each side of the centering axis, the two inclined faces having substantially symmetrical inclinations relative to the centering axis, the surgical tool resting on and between the two inclined faces of the support structure.

The support structure may be a V-shaped block. The two arms of the V-shaped block correspond to the two inclined faces of the support structure and the centering axis coincides with a bottom of the V-shaped block.

The support structure may comprise two rollers extending on each side of the centering axis, each roller defining a respective inclined face of the support structure, the two inclined faces being curved with variable inclinations.

The device may further comprise an axial stop system configured to prevent a translation of the surgical tool in a direction of the centering axis, wherein the axial stop system comprises two axial stops positioned at a distance from each other along the centering axis. The second winding surface may be located between the two axial stops. More specifically, the second winding surface may be positioned on a longitudinal sleeve of a friction element fixed to the surgical tool and located between the two axial stops.

The axial stop system may comprise a disjointed V-shaped block comprising two separated arms forming the two axial stops, the two separate arms having substantially symmetrical inclinations relative to an axis perpendicular to the centering axis.

The device may further comprise a fitting groove extending substantially linearly perpendicular to the centering axis, wherein the fitting groove is formed in the support structure and receives the rope to prevent a translation of the rope in a direction of the centering axis.

The first winding surface may comprise a circular groove receiving the rope and/or the second winding surface may comprise a circular groove receiving the rope.

The tensioning device may comprise a spring.

The drive system may comprise a knob rigidly secured to the shaft in rotation around the shaft axis, the knob being configured to be manually rotated by a user.

The drive system may comprise a motorized actuator configured to drive the shaft in rotation around the shaft axis.

The device according may comprise an additional rope looped around the first winding surface and the second winding surface, wherein the tensioning device tensions an additional loop made by the additional rope so that a rotation of the shaft results in simultaneous rotations of the rope and the additional rope and that simultaneous rotations of the rope and the additional rope result in a rotation of the surgical tool due to friction between the rope and the second winding surface and between the additional rope and the second winding surface.

The device for orienting a surgical tool may further comprise a support on which the shaft, drive system and tensioning device are mounted.

The surgical tool may be an endoscope.

According to a second aspect, the present disclosure relates to a tool positioning apparatus, comprising the device for orienting a surgical tool according to the first aspect and a device holder attached to the device for orienting a surgical tool by an attachment system, wherein the device holder is configured for positioning the device for orienting the surgical tool according to one to six degrees of freedom.

### BRIEF DESCRIPTION OF THE DRAWINGS

Other features and advantages of the disclosure will appear in the following description with reference to the drawings, in which:
Figure 1 illustrates a schematic perspective view of elements of a device for orienting a surgical tool according to an embodiment.
Figure 2 illustrates a schematic perspective view of a device for orienting a surgical tool according to an embodiment.
Figure 3 illustrates a schematic cross-sectional front view of the device of figure 2.
Figure 4 illustrates a schematic kinematic view of the device of figure 1.
Figure 5 illustrates a schematic cross-sectional front view of the device of figure 1.
Figure 6 illustrates a schematic perspective view of a first example of centering system of a device for orienting a surgical tool.
Figure 7 illustrates a schematic perspective view of a second example of centering system of a device for orienting a surgical tool.
Figure 8 illustrates a schematic side view of a device for orienting a surgical tool according to an embodiment.

### DETAILED DESCRIPTION

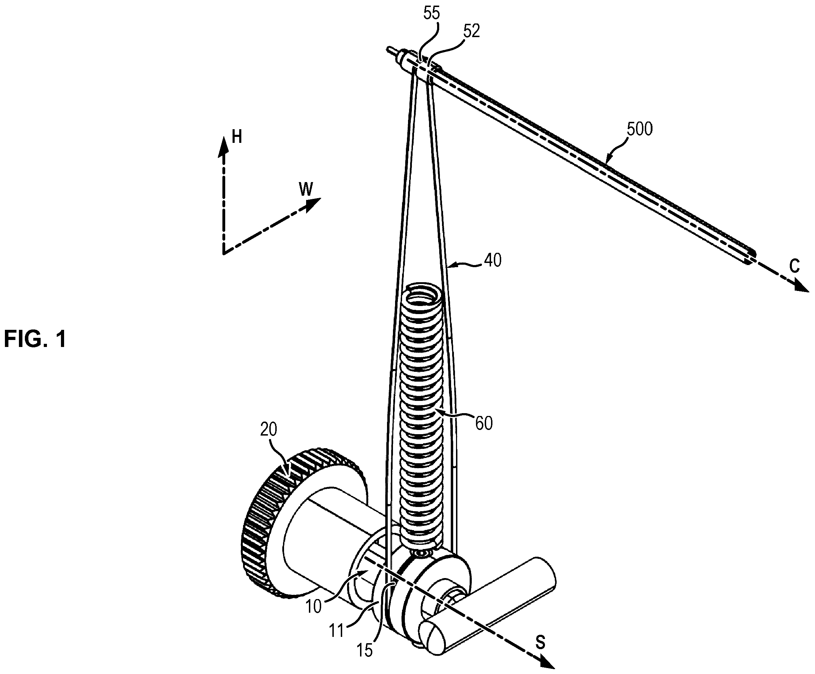

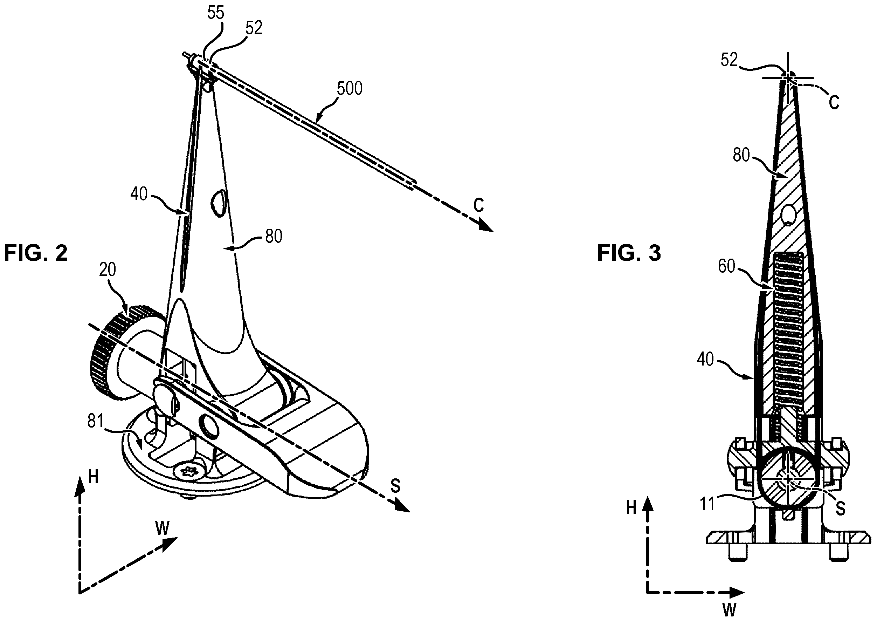

A device for orienting a surgical tool 500 is illustrated by way of a non-limiting example in figure 1 and in figure 2. The device comprises:
- a shaft 10 extending around a shaft axis S, the shaft 10 comprising a first winding surface 11;
- a drive system 20 configured to drive the shaft 10 in rotation around the shaft axis S;
- a surgical tool 500 rotationally coupled to a second winding surface 52;
- a rope 40 looped around the first winding surface 11 and the second winding surface 52; and
- a tensioning device tensioning the loop made by the rope 40 around the first winding surface 11 and the second winding surface 52, so that a rotation of the shaft 10 results in a rotation of the rope 40 and a rotation of the rope 40 results in a rotation of the second winding surface 52, thus of the surgical tool 500, due to a friction between the rope 40 and the second winding surface 52.

The above-disclosed device is a mechanical device which relies on friction to rotate the surgical tool 500 without needing any motors or electronic components and is therefore simple and cheap.

The above-mentioned device is also easy to use and necessitates fewer manipulations by the user to achieve the desired orientation of the surgical tool 500. In particular, the surgeon can first orient the surgical tool 500 thanks to the device, and then have both hands free to perform the surgery.

The above-mentioned device allows for positioning the surgical tool 500 in a wide variety of orientations, therefore is a flexible device which can be used in various surgical operations.

### Rope

The rope 40, first winding surface 11 and second winding surface 52 may all rotate in a same direction corresponding to the direction of the shaft axis S.

The rope 40 allows for transferring a rotational motion of the shaft 10, that is to say of the first winding surface 11, to the second winding surface 52, thus to the surgical tool 500. In other words, when the shaft 10 rotates, it rotates the rope 40, which then causes the surgical tool 500 to rotate.

The rope 40 forms a continuous loop between the first winding surface 11 and the second winding surface 52.

In the following, the term "height" is used in reference to a direction perpendicular to the direction of the shaft axis S and corresponding to a main direction of extension of the loop formed by the rope 40. The term "width" is used in reference to a lateral direction, or width direction W, which is perpendicular to the direction of the shaft axis S and to the height direction H. A width of the loop made by the rope 40 corresponds to a distance separating two sides of the loop made by the rope 40 at a given height.

The loop made by the rope 40 may extend along any path that allows the rope 40 to be wound around the first winding surface 11 and the second winding surface 52. The loop made by the rope 40 may extend along a path with round edges, a first round edge of the loop being formed around the first winding surface 11 and a second round edge of the loop being formed around the second winding surface 52.

When diameters of the first winding surface 11 and second winding surface 52 are substantially identical, the loop made by the rope 40 may extend along a rectangular path with round edges, the rope 40 extending substantially linearly in the height direction H between the two round edges.

When diameters of the first winding surface 11 and second winding surface 52 are different, the rope 40 may extend along a substantially conical path with rounded edges, the conical path narrowing toward the winding surface having the smaller diameter. Alternatively, a first part of the loop proximate the winding surface having the greater diameter may extend in a substantially rectangular path and a second part of the loop proximate the winding surface having the smaller diameter may extend in a substantially conical path, as illustrated by way of a non-limiting example in figure 3.

The term "rope 40" encompasses any kind of element which can be wound between the first winding surface 11 and second winding surface 52 to allow the transmission of the rotational motion of the first winding surface 11 to the second winding surface 52, for example may be a cable, a braided cable, a chain, a belt, etc.

The rope 40 may have any shape allowing the transmission of the rotational motion of the shaft 10 to the second winding surface 52, for example may be round, flat, V-shaped, with teeth configured to mesh with corresponding teeth of the first winding surface 11 and/or second winding surface 52, etc.

The rope 40 may be manufactured in any suitable materials, such as rubber, polyurethan, nylon, steel, cotton, etc.

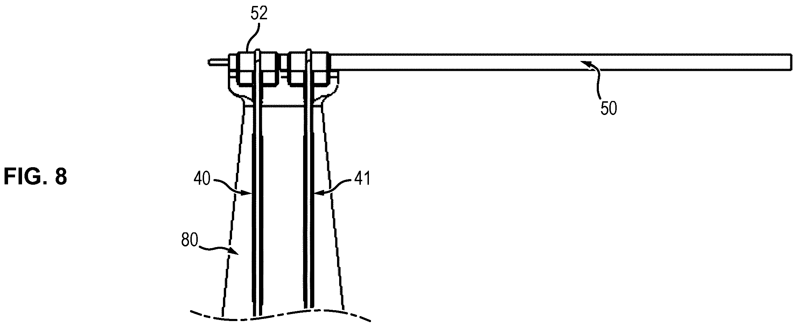

The device may comprise an additional rope 41 looped around the first winding surface 11 and the second winding surface 52, as illustrated by way of a non-limiting example in figure 8. The tensioning device tensions the additional loop made by the additional rope 41 so that a rotation of the shaft 10 results in simultaneous rotations of the rope 40 and the additional rope 41 and that simultaneous rotations of the rope 40 and the additional rope 41 result in a rotation of the second winding surface 52, thus of the surgical tool 500, due to friction between the rope 40 and the second winding surface 52 and between the additional rope 41 and the second winding surface 52. Such an additional rope 41 ensures more stable maintaining of the surgical tool 500 in position and better transmission of the rotation of the first winding surface 11 to the second winding surface 52, as it increases the number of ropes 40, 41, thus the friction between the ropes 40, 41 and the winding surfaces 11, 52.

Alternatively, the additional rope 41 may be looped around an additional first winding surface of the shaft 10 and an additional second winding surface rotationally coupled to the surgical tool 500. The additional first winding surface and the additional second winding surface may be similar to the first winding surface 11 and the second winding surface 52.

A same tensioning device may be used to tension the rope 40 and the additional rope 41. Alternatively, an additional tensioning device may be used to tension the additional rope 41, while the tensioning device is used to tension the rope 40. The rope 40 and additional rope 41 may be tensioned in a similar way by the tensioning system. Each of the rope 40 and additional rope 41 may cause substantially a similar amount of friction on the second winding surface 52 and if applicable on the additional second winding surface.

The rope 40 and additional rope 41 may be substantially identical and may extend in a similar configuration. The rope 40 and additional rope 41 may be positioned parallel to one another, one behind the other in the direction of the shaft axis S. The rope 40 and additional rope 41 may be positioned at a distance from one another in the direction of the shaft axis S allowing both the rope 40 and the additional rope 41 to be wound between the first winding surface 11 and the second winding surface 52. The two loops made by the rope 40 and the additional rope 41 may be substantially identical and positioned one behind the other along the shaft axis S. For example, the rope 40 can be positioned behind the additional rope 41 along the shaft axis S.

More than one additional rope 41 can be used.

### Winding surfaces

The first winding surface 11 and the second winding surface 52 may be separated from each other by a constant predetermined distance along the height direction H and may be positioned at a same position in the width direction W and at a same position in the direction of the shaft axis S.

The second winding surface 52 may be a surface of the surgical tool 500, for example may be part of an outer surface of the surgical tool 500. Alternatively, the second winding surface 52 may be a surface, for example an outer surface, of a friction element assembled to the surgical tool 500 so that the surgical tool 500 is rotationally fixed to the friction element. The second winding surface 52 may extend along a tool axis parallel to the shaft axis S.

The first winding surface 11 may be substantially a cylindrical surface of revolution around the shaft axis S, a length of the cylinder of the first winding surface 11 being directed along the shaft axis S. The second winding surface 52 may be substantially a cylindrical surface of revolution around the tool axis, a length of the cylinder of the second winding surface 52 being directed along the tool axis. The length of the first winding surface 51 may be substantially identical to the length of the second winding surface 52.

The first winding surface 11 and the second winding surface 52 may have substantially similar or different diameters. For example, the second winding surface 52 may have a smaller diameter than the first winding surface 11. The diameters of the first winding surface 11 and second winding surface 52 may be chosen according to a desired speed ratio between the rotation of the first winding surface 11 and the rotation of the second winding surface 52 and/or according to the rope 40 tension, the rope 40's speed of rotation, etc.

The first winding surface 11 may comprise a circular groove 15 receiving the rope 40 and/or the second winding surface 52 may comprise a circular groove 55 receiving the rope 40. The circular groove 15 of the first winding surface 11 is centered around the shaft axis S. The circular groove 55 of the second winding surface 52 is centered around the tool axis. These circular grooves 15, 55 allow maintaining the rope 40 in an optimal position and direction to ensure optimal transfer of the rotation of the first winding surface 11 to the second winding surface 52, the loop of the rope 40 extending mainly in the height direction H.

The circular grooves 15, 55 of the first winding surface 11 and/or of the second winding surface 52 may have any shape and/or dimensions allowing accommodation of the rope 40 and transmission of the rotation of the first winding surface 11 to the rope 40 and of the rope 40 to the second winding surface 52. For example, the circular grooves 15, 55 may be flat grooves if the rope 40 has a flat shape, V-grooves if the rope 40 has a V-shape, toothed grooves if the rope 40 has teeth, etc.

The circular groove 15 of the first winding surface 11 may be located substantially in a middle of the first winding surface 11 along the shaft axis S, the circular groove 15 dividing the first winding surface 11 into two substantially equal sections. The first winding surface 11 may extend substantially symmetrically on each side of the circular groove 15.

The circular groove 55 of the second winding surface 52 may be located substantially in a middle of the second winding surface 52 along the tool axis, the circular groove 55 dividing the second winding surface 52 into two substantially equal sections. The second winding surface 52 may extend substantially symmetrically on each side of the circular groove 55.

When the device comprises an additional rope 41, the first winding surface 11 may comprise an additional circular groove receiving the additional rope 41 and/or the second winding surface 52 may comprise an additional circular groove receiving the additional rope 41. The additional circular groove of the first winding surface 11 may be substantially identical to the circular groove of the first winding surface 11 and may be positioned in front of the circular groove of the first winding surface 11 along the shaft axis S. Similarly, the additional circular groove of the second winding surface 52 may be substantially identical to the circular groove of the second winding surface 52 and may be positioned in front of the circular groove of the second winding surface 52 along the tool axis.

### Centering system

The device may further comprise a centering system aligning the surgical tool 500 with a centering axis C parallel to the shaft axis S, wherein the surgical tool 500 is held between the centering system and the rope 40 due to a tension applied by the rope 40 on the surgical tool 500. Such a centering system allows the surgical tool 500 to rest on the centering system, if applicable via the friction element comprising the second winding surface 52, the friction element being positioned between the surgical tool 500 and the centering system. The surgical tool 500 is thus easily held in position by being sandwiched between the centering system below and the rope 40 above. Thus, the centering system allows for precise and easy centering of the surgical tool 500, so that the tool axis coincides with the centering axis C.

The centering axis C may be located at a same position in the width direction W as the shaft axis S, therefore the centering system positions the second winding surface 52 directly above the first winding surface 11 in the height direction H.

The centering system may comprise a support structure extending along the centering axis C, the support structure having two inclined faces located on each side of the centering axis C, the two inclined faces having substantially symmetrical inclinations relative to the centering axis C, the surgical tool 500, if applicable via the friction element, resting on and between the two inclined faces of the support structure. Such a support structure having two inclined faces allows for a precise and simple mechanical centering of the surgical tool 500, which ensures that the surgical tool 500 remains aligned with the centering axis C without necessitating any action from the user.

The two inclined faces are positioned at a same height, laterally on each side of the centering axis C. The inclinations of the two inclined faces may be constant or variable inclinations. The two inclined faces may extend along similar dimensions in the centering axis C as the second winding surface 52 extends along the tool axis. The two inclined faces may be positioned at identical positions along the centering axis C.

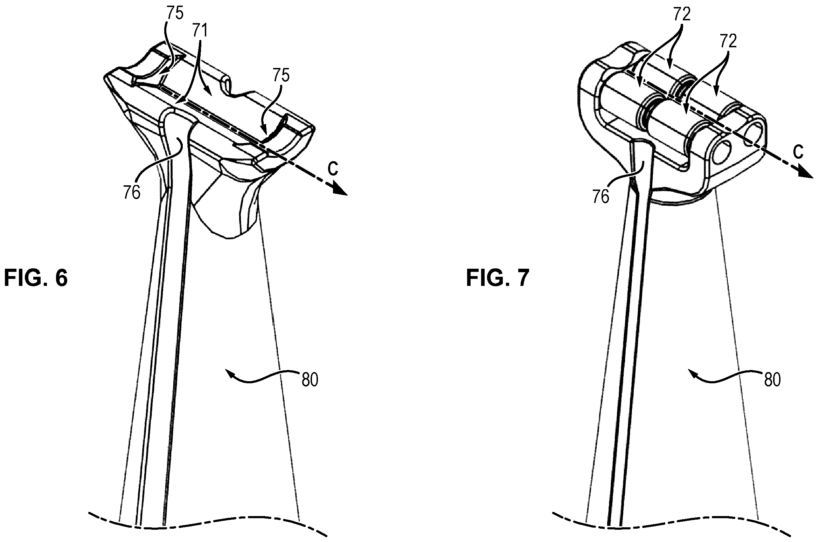

In a first embodiment, illustrated by way of a non-limiting example in figure 6, the support structure is a V-shaped block. The two arms 71 of the V-shaped block correspond to the two inclined faces of the support structure and the centering axis C coincides with a bottom of the V-shaped block. Such a V-shaped block is particularly easy and cheap to manufacture.

Each arm 71 of the V-shaped block may be a substantially planar surface having a substantially constant inclination relative to the centering axis C. Alternatively, each arm 71 of the V-shaped block may be a substantially curved surface having a variable inclination relative to the centering axis C. The inclination of each arm 71 of the V-shaped block relative to the centering axis C may be comprised between 5° and 45°, for example may be comprised between 10° and 20°.

In a second embodiment, illustrated by way of a non-limiting example in figure 7, the support structure comprises two rollers 72 extending on each side of the centering axis C. Each roller 72 defines a respective inclined face of the support structure, the two inclined faces being curved with variable inclinations. Such rollers 72 allow a particularly stable centering of the surgical tool 500 and fluid rotation of the second winding surface 52, thus of the surgical tool 500.

The two rollers 72 may be located adjacent to each other, side by side in the lateral direction, and at identical positions along the centering axis C. The two rollers 72 may be identical, especially may have identical diameters. The diameters of the two rollers 72 may be adjusted in view of a dimension of the second winding surface 52. Each roller 72 may be rotatable around a roller shaft parallel to the centering axis C, the two roller shafts being spaced apart in the width direction Wand located at identical heights. A direction of rotation of the two rollers 72 may be opposed to the direction of rotation of the first winding surface 11, second winding surface 52 and rope 40.

The support structure may comprise two pairs of two rollers 72 as disclosed above. The two pairs are positioned one behind the other in the direction of the centering axis C. Two rollers 72 located on a same side of the centering axis C may be mounted on the same roller shaft. A space may be arranged between the two pairs so as to accommodate the rope 40 between the two pairs. More specifically, each side of the loop formed by the rope 40 may be accommodated between a front roller 72 and a back roller 72 on the same side of the centering axis C. Thus, the rollers 72 participate in maintaining the rope 40 in position along the centering axis C.

When the device comprises an additional rope 41, the rope 40 may be positioned on the rear pair of rollers 72 and the additional rope 41 may be positioned on the front pair of rollers 72.

The device may further comprise a fitting groove 76 extending substantially linearly perpendicular to the centering axis C, wherein the fitting groove 76 is formed in the support structure and receives the rope 40 to prevent a translation of the rope 40 in a direction of the centering axis C. The fitting groove 76 may extend mainly in the height direction H. The fitting groove 76 allows maintaining the rope 40 in an optimal position and direction to ensure optimal transfer of the rotation of the first winding surface 11 to the second winding surface 52 and optimal positioning of the surgical tool 500, the loop of the rope 40 extending mainly in the height direction H.

The fitting groove 76 may have any shape and/or dimensions allowing accommodation of the rope 40 and rotation of the rope 40. More specifically, the fitting groove 76 may be flat, V-shaped, etc.

The fitting groove 76 may be located substantially in a middle of the support structure along the centering axis C, the fitting groove 76 dividing the support structure into two substantially equal sections. The support structure may extend substantially symmetrically on each side of the fitting groove 76.

The device may comprise two such fitting grooves 76, positioned symmetrically with respect to the centering axis C, on each side of the support structure in the width direction W. Thus, each side of the loop made by the rope 40 is received in a respective fitting groove 76 located on a respective side of the support structure, allowing a better guiding of the rotation of the rope 40.

When the device comprises an additional rope 41, each side of the rope 40 may be positioned in a fitting groove 76 on a respective side of the support structure and similarly, each side of the additional rope 41 may be positioned in an additional fitting groove on a respective side of the support 80, each additional fitting groove being located in front of each corresponding fitting groove 76.

### Surgical tool

The surgical tool 500 may comprise a body. The body may extend at least partly cylindrically along the tool axis. The second winding surface 52 may correspond to an outer surface of the cylindrical part of the body of the surgical tool 500.

The surgical tool 500 may be an endoscope, such as a standard tubular endoscope. The device for orienting a surgical tool 500 is then an endoscope holder. The device then allows the practitioner to access a wide field of view by rotating the endoscope or videoscope in the appropriate position.

### Friction element

The device may comprise a friction element assembled to the surgical tool 500 so that the surgical tool 500 is rotationally fixed to the friction element. The surgical tool 500 may also be translationally fixed to the friction element, the friction element being attached to and moving with the surgical tool 500. The friction element may comprise the second winding surface 52. The second winding surface 52 may be an outer surface of the friction element.

The friction element may be a longitudinal sleeve attached around the surgical tool 500, for example attached to the body of the surgical tool 500. The longitudinal sleeve protrudes radially from the body of the surgical tool 500.

The longitudinal sleeve of the friction element may be a cylindrical sleeve positioned around the body of the surgical tool 500, for example around a cylindrical part of the body of the surgical tool 500. When the body of the surgical tool 500 is cylindrical, the longitudinal sleeve has a greater diameter than a diameter of the body of the surgical tool 500.

The friction element is configured to rest on the support structure. The friction element may have a dimension along the tool axis corresponding substantially to a dimension of the support structure along the centering axis C. Therefore, the whole friction element can rest on the support structure, while no part of the surgical tool 500 rests on the support structure.

### Axial stop system

The device may further comprise an axial stop system configured to prevent a translation of the surgical tool 500 in a direction of the centering axis C, wherein the axial stop system comprises two axial stops 75 positioned at a distance from each other along the centering axis C. The second winding surface 52 may be located between the two axial stops and may abut the two axial stops in order to prevent a translation of the surgical tool 500 in the direction of the centering axis C.

For example, the second winding surface 52 of the surgical tool 500 may abut the two axial stops. Alternatively, the second winding surface 52 may be positioned on the longitudinal sleeve of the friction element fixed to the surgical tool 50 and located between the two axial stops 75.

Thus, the axial stop system allows for precise positioning of the surgical tool 500 along the centering axis C, in addition to the centering along the centering axis C. Therefore, the axial stop system ensures that the first winding surface 11 and second winding surface 52 always remain facing each other at substantially a same position along the centering axis C. Therefore, the loop made by the rope 40 remains substantially directed along the height direction H.

The longitudinal sleeve of the friction element can come directly to a stop against the axial stops 75, at a front end and at a rear end of the longitudinal sleeve, in order to prevent the translation of the surgical tool 500 along the centering axis C, the surgical tool 500 and longitudinal sleeve being translationally coupled. The two axial stops 75 are then separated approximately by a dimension of the longitudinal sleeve along the centering axis C. The two axial stops 75 may be positioned at a rear end of the support structure and at a front end of the support structure.

The axial stop system may comprise a disjointed V-shaped block comprising two separated arms forming the two axial stops 75, the two separate arms having substantially symmetrical inclinations relative to an axis perpendicular to the centering axis C, that is to say relative to an axis directed along the height direction H. Such a disjointed V-shaped block, for example cooperating with the longitudinal sleeve of the friction element, is an efficient and simple way to achieve the positioning of the surgical tool 500 along the centering axis C.

A rear arm of the disjointed V-shaped block may be positioned at a rear end of the support structure and a front arm of the disjointed V-shaped block may be positioned at a front end of the support structure. The rear arm and the front arm of the disjointed V-shaped block may be arranged symmetrically relative to an axis oriented along the height direction H.

The inclination of each arm of the disjointed V-shaped block relative to the axis oriented along the height direction H may be comprised between 0° and 90°, for example may be comprised between 20° and 60°, for example may be equal to 45°. An angle of 0° means that each arm of the V-shaped block is oriented along the height direction H.

### Tensioning device

The tensioning device sets a tension of the rope 40 wound between the first winding surface 11 and the second winding surface 52, that is to say tightened around the first winding surface 11 and the second winding surface 52. The tension of the rope 40 is chosen in order to ensure enough friction between the first winding surface 11 and the second winding surface 52 to transmit the rotation, and also to help maintain the rope 40 in place on the first winding surface 11 and the second winding surface 52. However, the tension of the rope 40 is kept low enough to reduce a wear on the rope 40, the first winding surface 11 and the second winding surface 52.

The tensioning device may be configured to apply a force to the shaft 10 so as to change a position of the shaft 10 along the height direction H. A distance between the shaft 10 and the surgical tool 500 in the height direction H is thus adapted as a consequence of the force applied by the tensioning device, in order to maintain an appropriate tension of the loop formed by the rope 40. More specifically, the tensioning device may be configured to apply a steady downward force to the shaft 10.

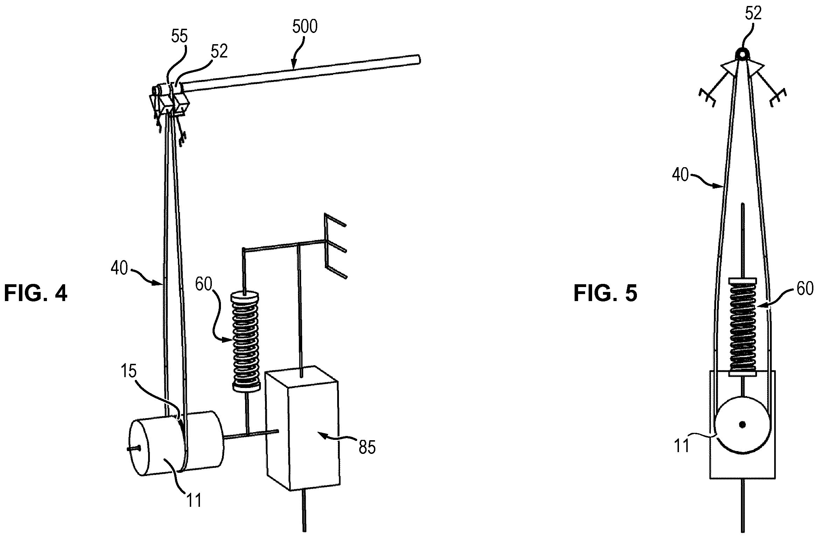

The tensioning device may comprise any device configured to tension the rope 40, for example may comprise a counterweight tensioner or an eccentric tensioner. More particularly, the tensioning device may comprise a spring 60 extending substantially in the height direction H, as illustrated by way of a non-limiting example in figure 3 and in figure 5. A spring 60 is a simple mechanical means for tensioning the rope 40. Characteristics of the spring 60, such as the stiffness, deflection range, material, etc., of the spring 60, may be chosen to achieve optimal tensioning of the rope 40.

The spring 60 may be a compression spring 60.

The compression spring 60 may comprise a first end mounted on the shaft 10 and a second end mounted on a support 80 of the device for orienting the surgical tool 500. The compression spring 60 applies the tension to the first shaft 10, more specifically may apply a steady downward force to the first shaft 10 in order to keep the rope 40 tensioned between the shaft 10 and the tool.

The compression spring 60 may be compressed to loosen the rope 40, which allows freeing the surgical tool 500. The surgical tool 500 can then be removed, for example to be stored or replaced by another surgical tool 500.

### Shaft

The shaft 10 may be cylindrical around the shaft axis S. The shaft 10 may comprise a longitudinal sleeve attached to the shaft 10. An outer surface of the longitudinal sleeve forms the first winding surface 11. The longitudinal sleeve protrudes radially from the shaft 10.

The longitudinal sleeve may be a cylindrical sleeve positioned around the cylindrical shaft 10, the longitudinal sleeve having a greater diameter than a diameter of the shaft 10.

The longitudinal sleeve of the shaft 10 may have a dimension along the shaft axis S corresponding substantially to a dimension of the longitudinal sleeve of the friction element along the tool axis. Therefore, the longitudinal sleeve of the shaft 10 and the longitudinal sleeve of the friction element may positioned at identical positions along the shaft axis S, so the rope 40 extends perpendicularly to the shaft axis S.

The shaft 10 may comprise a rear end and a front end opposite the rear end along the shaft axis S. The front end of the shaft 10 may be mounted in a sliding joint 85 directed along a height direction H, as illustrated by way of a non-limiting example in figure 4. Therefore, the position of the shaft 10 along the height direction H can be modified by the tensioning device to maintain a substantially constant tension to the rope 40.

### Drive system

The drive system 20 may be any kind of drive system which drives the shaft 10 in rotation around the shaft axis S when actuated.

The drive system 20 may comprise a knob rigidly secured to the shaft 10 in rotation around the shaft axis S, the knob being configured to be manually rotated by a user. Such a knob is simple, ergonomic, and easy to use with minimal manipulation. The rotation applied to the knob by the user is directly and mechanically transmitted to the shaft 10.

The knob may be secured to the rear end of the shaft 10, so that it is easily accessible to be rotated by the user. The knob may have a shape of a cylinder extending along the shaft axis S and secured around the shaft 10, the knob having a greater diameter than the diameter of the shaft 10.

Alternatively or in addition, the drive system 20 may comprise a motorized actuator configured to drive the shaft 10 in rotation around the shaft axis S. Such a motorized actuator may offer high precision in the command of the rotation and may easily be actuated by the user with minimal manipulation.

The motorized actuator may be an electric or a hydraulic actuator.

### Support

The device for orienting a surgical tool 500 may further comprise a support 80 on which the shaft 10, drive system 20 and tensioning device are mounted. Such a support 80 is illustrated by way of a non-limiting example in figure 2.

An upper end of the support 80 may be formed by the centering system, more specifically by the support structure of the centering system. An upper part of the body extends as a continuation of the support structure.

A body of the support 80 may extend from the upper end to a lower end opposite the upper end in the height direction H.

The body of the support 80, for example the upper part of the body, may have a conical shape which narrows towards the upper end of the support 80. Such a conical shape is particularly appropriate when the first winding surface 11 has a greater diameter than the second winding surface 52, as the loop made by the rope 40 then has at least partially a conical shape. For example, the rope 40 may rest on each side of the upper part of the body, the loop made by the rope 40 having a conical shape proximate the second winding surface 52. Proximate the first winding surface 11, the rope 40 may be accommodated inside the body of the support 80, the loop made by the rope 40 having a rectangular shape. Each side of the outer face of the body of the support 80 is then pierced with a hole configured to allow the rope 40 to pass through.

The fitting groove 76 may extend in the support structure and in the upper part of the body of the support 80, more specifically in the outer surface of the upper part of the body. The fitting groove 76 made in the conical upper part of the body allows guiding the shape and rotation of the conical part of the loop.

The tensioning device may be mounted inside the body of the support 80.

The shaft 10 may be mounted to have an end extending from the body of the support 80, in order for the drive system 20 to be easily accessible to a user. The first winding surface 11 may be located inside the body of the support 80.

The lower end of the support 80 may be configured to be attached to a device holder. The lower end of the support 80 may for example be a plate 81 extending substantially perpendicular to the height direction H.

### Tool positioning apparatus

A tool positioning apparatus may comprise the device for orienting a surgical tool 500 as disclosed above and a device holder attached to the device for orienting a surgical tool 500 by an attachment system. The device holder may be configured for positioning the device for orienting the surgical tool 500 according to one to six degrees of freedom, for example according to up to three degrees of freedom in translation and up to three degrees of freedom in rotation.

Such a tool positioning apparatus presents allows positioning the device for orienting a surgical tool 500 in wide variety of positions and/or orientations, thanks to the device holder.

The tool positioning apparatus may be mounted on the lower end of the support 80, for example on the plate 81 forming the lower end of the support 80.

The device holder may for example be a robotic system, for example a motorized arm of a robotic system.

Obviously, the present disclosure cannot be limited to the means and configuration described and illustrated herein, and it also extends to any equivalent means or configurations and to any technically operative combination of such means.

## Claims

1. A device for orienting a surgical tool (500), comprising:
- a shaft (10) extending around a shaft axis (S), the shaft (10) comprising a first winding surface (11);
- a drive system (20) configured to drive the shaft (10) in rotation around the shaft axis (S);
- a surgical tool (500) rotationally coupled to a second winding surface (52);
- a rope (40) looped around the first winding surface (11) and the second winding surface (52); and
- a tensioning device tensioning the loop made by the rope (40) around the first winding surface (11) and the second winding surface (52), so that a rotation of the shaft (10) results in a rotation of the rope (40) and a rotation of the rope (40) results in a rotation of the second winding surface (52), thus of the surgical tool (500), due to a friction between the rope (40) and the second winding surface (52).

2. The device according to claim 1, further comprising a centering system aligning the surgical tool (500) with a centering axis (C) parallel to the shaft axis (S), wherein the surgical tool (500) is held between the centering system and the rope (40) due to a tension applied by the rope (40) on the surgical tool (500).

3. The device according to claim 2, wherein the centering system comprises a support structure extending along the centering axis (C), the support structure having two inclined faces located on each side of the centering axis (C), the two inclined faces having substantially symmetrical inclinations relative to the centering axis (C), the surgical tool (500) resting on and between the two inclined faces of the support structure.

4. The device according to claim 3, wherein the support structure is a V-shaped block, the two arms (71) of the V-shaped block corresponding to the two inclined faces of the support structure and the centering axis (C) coinciding with a bottom of the V-shaped block.

5. The device according to claim 3, wherein the support structure comprises two rollers (72) extending on each side of the centering axis (C), each roller (72) defining a respective inclined face of the support structure, the two inclined faces being curved with variable inclinations.

6. The device according to any one of claims 2 to 5, further comprising an axial stop system configured to prevent a translation of the surgical tool (500) in a direction of the centering axis (C), wherein the axial stop system comprises two axial stops (75) positioned at a distance from each other along the centering axis (C), wherein the second winding surface (52) is located between the two axial stops (75).

7. The device according to claim 6, wherein the axial stop system comprises a disjointed V-shaped block comprising two separated arms forming the two axial stops (75), the two separate arms having substantially symmetrical inclinations relative to an axis perpendicular to the centering axis (C).

8. The device according to any one of claims 3 to 5, further comprising a fitting groove (76) extending substantially linearly perpendicular to the centering axis (C), wherein the fitting groove (76) is formed in the support structure and receives the rope (40) to prevent a translation of the rope (40) in a direction of the centering axis (S).

9. The device according to any one of claims 1 to 8, wherein the first winding surface (11) comprises a circular groove (15) receiving the rope (40) and/or the second winding surface (52) comprises a circular groove (55) receiving the rope (40).

10. The device according to any one of claims 1 to 9, wherein the tensioning device comprises a spring (60).

11. The device according to any one of claims 1 to 10, wherein the drive system (20) comprises a knob rigidly secured to the shaft (10) in rotation around the shaft axis (S), the knob being configured to be manually rotated by a user.

12. The device according to any one of claims 1 to 11, wherein the drive system (20) comprises a motorized actuator configured to drive the shaft (10) in rotation around the shaft axis (S).

13. The device according to any one of claims 1 to 12, comprising an additional rope (41) looped around the first winding surface (11) and the second winding surface (52), wherein the tensioning device tensions an additional loop made by the additional rope (41) so that a rotation of the shaft (10) results in simultaneous rotations of the rope (40) and the additional rope (41) and that simultaneous rotations of the rope (40) and the additional rope (41) result in a rotation of the surgical tool (500) due to friction between the rope (40) and the second winding surface (52) and between the additional rope (41) and the second winding surface (52).

14. The device according to any one of claims 1 to 13, wherein the surgical tool (500) is an endoscope.

15. A tool positioning apparatus, comprising the device for orienting a surgical tool (500) according to any one of claims 1 to 14 and a device holder attached to the device for orienting a surgical tool (500) by an attachment system, wherein the device holder is configured for positioning the device for orienting the surgical tool (500) according to one to six degrees of freedom.
